# EUROPEAN PATENT APPLICATION

(11) **EP 4 549 568 A1**
(43) Date of publication of application: **07.05.2025**
(21) Application number: 23831225.0
(22) Date of filing: 21.06.2023
(51) Int. Cl.: C12N 15/113, A61K 31/7105, A61K 31/712, A61K 31/7125, A61K 48/00, A61P 35/00, C12N 5/09, C12N 5/10

(54) **SIRNA TARGETING RECQL1 HELICASE GENE**

(30) Priority: 30.06.2022 JP 2022105409
(71) Applicant: Genecare Research Institute Co., Ltd., Kamakura-shi, Kanagawa 248-0022 (JP); Solasia Pharma K.K., Tokyo 105-0011 (JP); The University of Tokyo, Bunkyo-ku, Tokyo 113-8654 (JP)
(72) Inventor: YAMADA Kayoko, Kamakura-shi, Kanagawa 248-0022 (JP); ROKUGAWA Kyuji, Kamakura-shi, Kanagawa 248-0022 (JP); TAKAHASHI Naoya, Kamakura-shi, Kanagawa 248-0022 (JP); UI-TEI Kumiko, Tokyo 113-8654 (JP); KOBAYASHI Yoshiaki, Tokyo 113-8654 (JP)
(74) Representative: J A Kemp LLP
(86) International application number: PCT/JP2023/022928
(87) International publication number: WO 2024/004779

(57) **Abstract**

It is an object of the present invention to provide an siRNA targeting RecQL1 helicase gene, based on a new target sequence that results in an excellent effect superior to that of conventional siRNAs.

Provided is an siRNA targeting RecQL1 helicase gene, consisting of a sense strand comprising the base sequence of SEQ ID NO: 1 and an antisense strand comprising the base sequence of SEQ ID NO: 2.

## Description

### Technical Field

The present invention relates to: an siRNA targeting RecQL1 helicase gene, an agent for treating cancer, and a pharmaceutical composition for treating cancer.

### Background Art

DNA helicase is an enzyme having an activity of dissociating a double-stranded DNA to single strands, and there are a variety of types of DNA helicases, and a DNA helicase similar to the RecQ helicase derived from *Escherichia coli* is collectively referred to as a "RecQ type helicase." In humans, five types of RecQ-type helicases (RecQL1, WRN, RTS, BLM, and RecQ5) are known.

The specific functions of the RecQL1 helicase (also referred to as RecQ1 or RecQL) are known to unwind the higher-order structure of DNA which is referred to as a holiday structure, upon genome replication, and to be associated with mismatch repair. Moreover, it has been known that the RecQL1 helicase is highly expressed in actively proliferating cells such as cancer cells, but that the expression level thereof is low in cells at resting phase.

The present inventors have developed, based on the above-described facts, an siRNA targeting the RecQL1 helicase as an agent for treating cancer. This siRNA has an activity of specifically inducing mitotic catastrophe and mitotic cell death to cancer cells (Patent Literature 1 to 3 and Non Patent Literature 1 to 3). It is considered that this is caused by the phenomenon whereby the function of the RecQL1 helicase is inhibited in cancer cells, thereby directly proceeding to cell division with the DNA higher-order structure and DNA damages that developed upon DNA replication, remaining in the cells, resulting in induction of cell death due to abnormalities in chromosome segregation and the like. The present inventors also found that the above-described siRNA shows an antitumor activity in cancer-bearing model animals and have revealed that the RecQL1 helicase can be an excellent target for treatment.

Patent Literature 3 discloses that RNAi activity based on an siRNA targeting a RecQL1 helicase gene is enhanced by introduction of chemical modifications. The siRNA described in Patent Literature 3 targets the base sequence corresponding to that at positions 784 to 802 in the mRNA of a human RecQL1 helicase gene (NCBI accession number NM_002907.4). This target sequence is the same as the siRNA target sequence disclosed in Patent Literature 1, and is a sequence selected based on the highest overall evaluation, including e.g., efficacy, in the previous research.

In the RecQL1 helicase gene, no target sequence has been found that can achieve an effect superior to that of the above-described target sequences.

### Citation List

### Patent Literature

Patent Literature 1: International Publication WO 2004/100990
Patent Literature 2: International Publication WO 2006/054625
Patent Literature 3: International Publication WO 2017/022650

### Non Patent Literature

Non Patent Literature 1: Futami, K., et al. (2008) Cancer Sci., 99(1): 71-80
Non Patent Literature 2: Futami, K., et al. (2008) Cancer Sci., 99(6): 1227-1236
Non Patent Literature 3: Futami, K., et al. (2010) Int. J. Mol. Med., 25: 537-545

### Summary of Invention

### Technical Problem

It is an object of the present invention to provide an siRNA targeting a RecQL1 helicase gene, based on a new target sequence that results in an excellent effect superior to that of conventional siRNAs.

### Solution to Problem

In order to solve the above-described problems, the present inventors have searched for a new siRNA target sequence that can exhibit properties superior to those of conventional siRNAs. The present inventors have found as a result thereof an siRNA based on a novel target sequence, having properties far superior to those of conventional siRNAs in terms of RNAi activity against cancer cells, low toxicity to normal cells, and stability in human serum. Furthermore, when the siRNA of the present invention was administered to a mouse model of peritoneal dissemination of human-derived ovarian cancer cell line ES-2, it was found to exhibit a high therapeutic effect on tumor cells in vivo. The present invention is based on the above findings and provides the following.
[1] An siRNA targeting a RecQL1 helicase gene, consisting of:
   a sense strand comprising the base sequence of SEQ ID NO: 1, and
   an antisense strand comprising the base sequence of SEQ ID NO: 2.
[2] The siRNA according to the above [1], comprising a natural ribonucleoside, a natural deoxyribonucleoside, and/or a modified nucleoside.
[3] The siRNA according to the above [2], wherein said modified nucleoside is a 2'-modified nucleoside and/or a bridged nucleoside.
[4] The siRNA according to the above [3], wherein the 2'-modification group of said 2'-modified nucleoside is a 2'-O-methyl group or a 2'-fluoro group.
[5] The siRNA according to any one of the above [1] to [4], wherein all or part of the internucleoside linkages in said sense strand and/or said antisense strand are a modified internucleoside linkage.
[6] The siRNA according to any one of the above [1] to [5], wherein said antisense strand comprises a 2'-modified nucleoside at position 2 in the base sequence of SEQ ID NO: 2.
[7] The siRNA according to any one of the above [1] to [6], wherein the nucleoside at position 14 in the base sequence of SEQ ID NO: 2 is unmodified in said antisense strand.
[8] The siRNA according to any one of the above [1] to [7], wherein said sense strand and/or said antisense strand comprises a 2'-modified nucleoside or a bridged nucleoside at the 3' end.
[9] The siRNA according to any one of the above [1] to [8], wherein:
   in said sense strand, the nucleoside at position 7, position 9, and/or position 11 in the base sequence of SEQ ID NO: 1 is unmodified, or is a 2'-fluoro-modified nucleoside; and/or
   in said antisense strand, the nucleoside at position 6 and/or position 12 in the base sequence of SEQ ID NO: 2 is unmodified, or is a 2'-fluoro-modified nucleoside, and/or the nucleoside at position 7 in the base sequence of SEQ ID NO: 2 is a 2'-O-methyl-modified nucleoside.
[10] The siRNA according to any one of the above [1] to [9], wherein said sense strand comprises a 2'-modified nucleoside at one or more positions at positions 9 to 11 in the base sequence of SEQ ID NO: 1, or said antisense strand comprises a 2'-modified nucleoside at one or more positions at positions 9 to 11 in the base sequence of SEQ ID NO: 2.
[11] The siRNA according to the above [1], wherein said sense strand and said antisense strand respectively consist of:
   (a) the base sequence of SEQ ID NO: 3 and the base sequence of SEQ ID NO: 4,
   (b) the base sequence of SEQ ID NO: 5 and the base sequence of SEQ ID NO: 6,
   (c) the base sequence of SEQ ID NO: 7 and the base sequence of SEQ ID NO: 8,
   (d) the base sequence of SEQ ID NO: 9 and the base sequence of SEQ ID NO: 10, or
   (e) the base sequence of SEQ ID NO: 11 and the base sequence of SEQ ID NO: 12.
[12] The siRNA of the above [1], wherein each of said sense strand and said antisense strand consists of natural ribonucleosides linked by internucleoside linkages.
[13] An agent for inducing cell death, comprising the siRNA according to any one of the above [1] to [12] as an active ingredient.
[14] An agent for treating cancer, comprising the siRNA according to any one of the above [1] to [12] as an active ingredient.
[15] A pharmaceutical composition for treating cancer, comprising the siRNA according to any one of the above [1] to [12].
[16] The pharmaceutical composition for treating cancer according to the above [15], wherein said cancer is ovarian cancer, breast cancer, melanoma, gastric cancer, pancreatic cancer, liver cancer, colorectal cancer, lung cancer, head and neck cancer, peritoneal cancer, or cervical cancer.

The present description includes part or all of the contents as disclosed in Japanese Patent Application No. 2022-105409, which is a priority document of the present application.

### Advantageous Effects of Invention

According to the present invention, provided is an siRNA targeting a RecQL1 helicase gene, based on a new target sequence that results in an excellent effect superior to that of the conventional siRNAs.

### Brief Description of Drawings

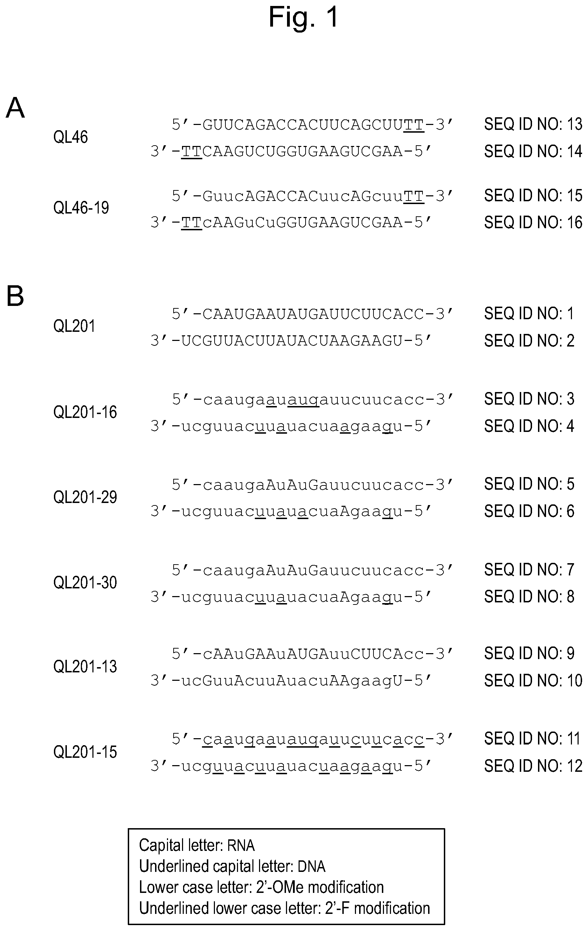

[Figure 1] Figure 1 is a view showing the structure of the siRNA prepared in Example. Figure 1A shows QL46, which is a conventional unmodified siRNA, and QL46-19, which is a conventional modified siRNA. Figure 1B shows QL201, which is the unmodified siRNA of the present invention, and QL201-16, QL201-29, QL201-30, QL201-13, and QL201-15, which are the modified siRNAs of the present invention.

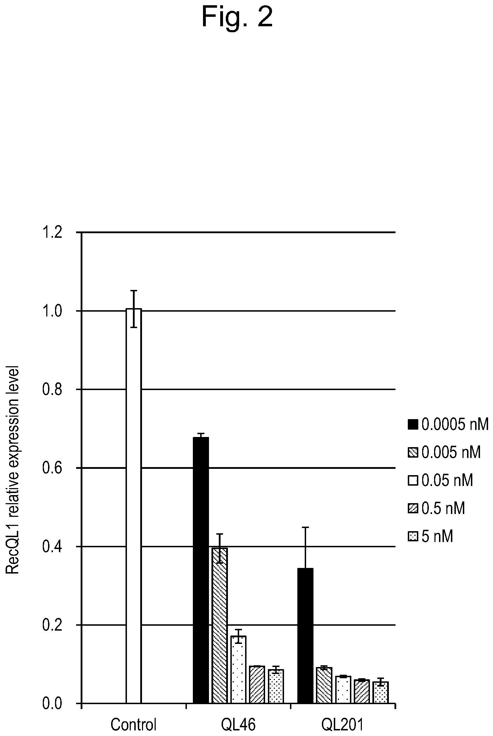

[Figure 2] Figure 2 is a graph showing the measurement results of RNAi activity to a RecQL1 gene in ES-2 cells. The results are shown when QL46, which was the conventional unmodified siRNA and QL201, which was the unmodified siRNA of the present invention were introduced into ES-2 cells at concentrations of 0.0005 nM to 5 nM. The error bars indicate standard deviation.

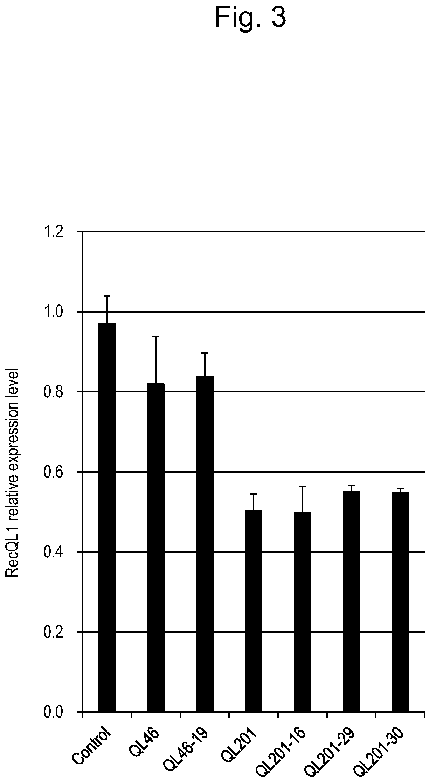

[Figure 3] Figure 3 is a graph showing the measurement results of RNAi activity to a RecQL1 gene in HeLa cells. Each siRNA was introduced into HeLa cells at a concentration of 0.005 nM. The error bars indicate standard deviation.

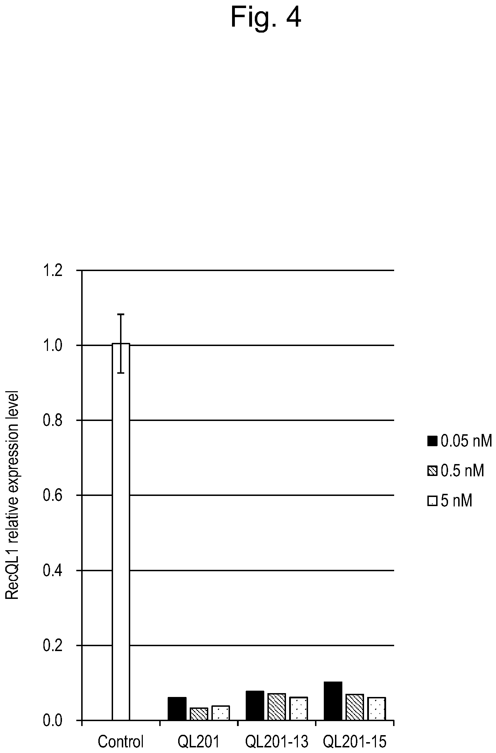

[Figure 4] Figure 4 is a graph showing the measurement results of RNAi activity to a RecQL1 gene in ES-2 cells. Each siRNA was introduced into ES-2 cells at concentrations of 0.05 nM to 5 nM. The error bars indicate standard deviation.

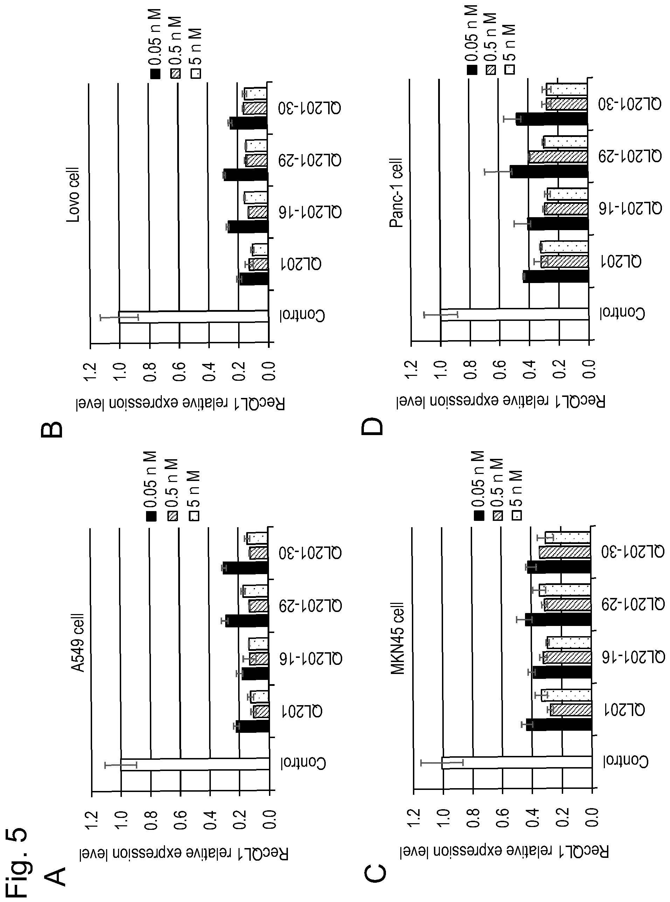

[Figure 5] Figure 5 illustrates graphs showing the measurement results of RNAi activity by the siRNA of the present invention in a variety of cancer cell lines. Figure 5A shows the results for A549 cells. Figure 5B shows the results for Lovo cells. Figure 5C shows the results for MKN45 cells, and Figure 5D shows the results for Panc-1 cells. The error bars indicate standard deviation.

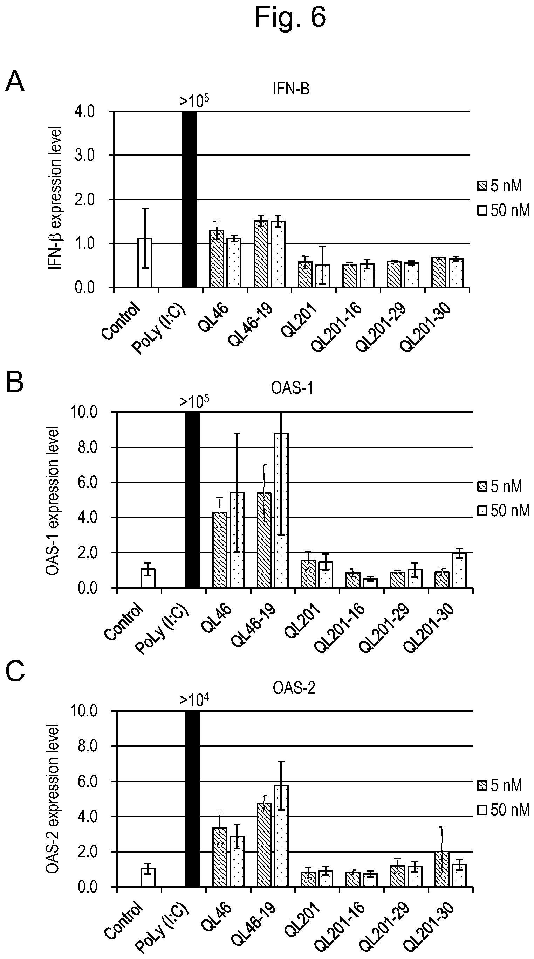

[Figure 6] Figure 6 illustrates graphs showing the measurement results of the immune responsiveness of the siRNA in a TIG-3 cell line. Figure 6A shows the measurement results of the expression level of the IFN-β gene. Figure 6B shows the measurement results of the expression level of the OAS-1 gene. Figure 6C shows the measurement results of the expression level of the OAS-2 gene. The error bars indicate standard deviation.

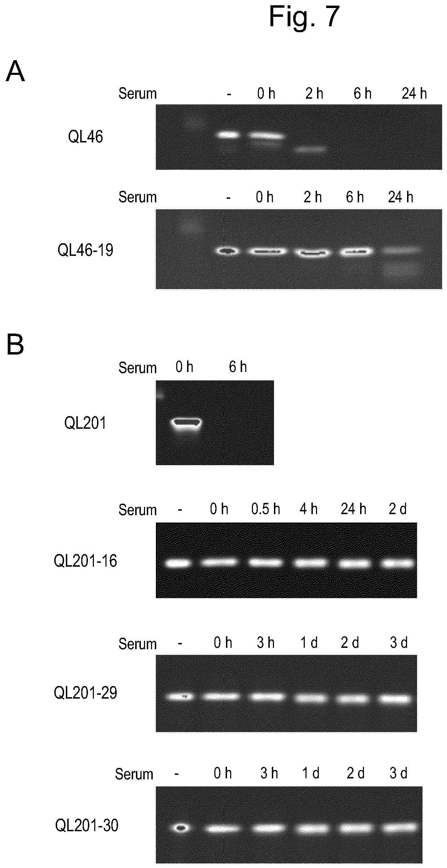

[Figure 7] Figure 7 is a view showing the results of electrophoresis of the siRNA after incubation in human serum. Figure 7A shows the results for QL46 and QL46-19. Figure 7B shows the results for QL201, QL201-16, QL201-29, and QL201-30. The black areas within each band in Figure 7A and Figure 7B indicate the presence of an undegraded siRNA beyond the upper limit of detection sensitivity.

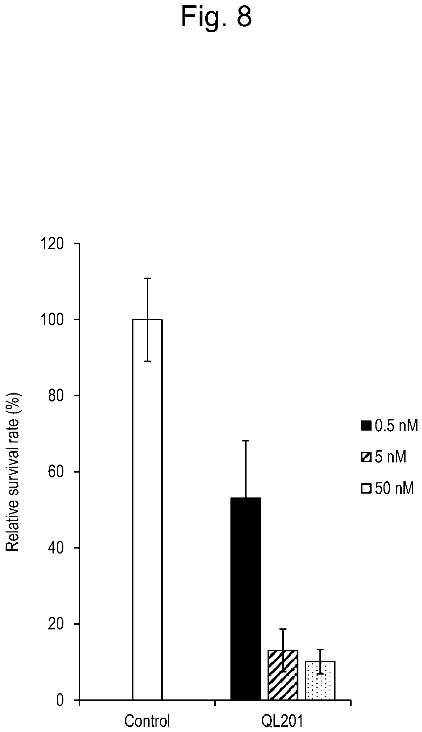

[Figure 8] Figure 8 is a graph showing the cell death-inducing activity of QL201 in cancer cells. The relative survival rate of ES-2 cells after introduction of QL201 is shown. The error bars indicate standard deviation.

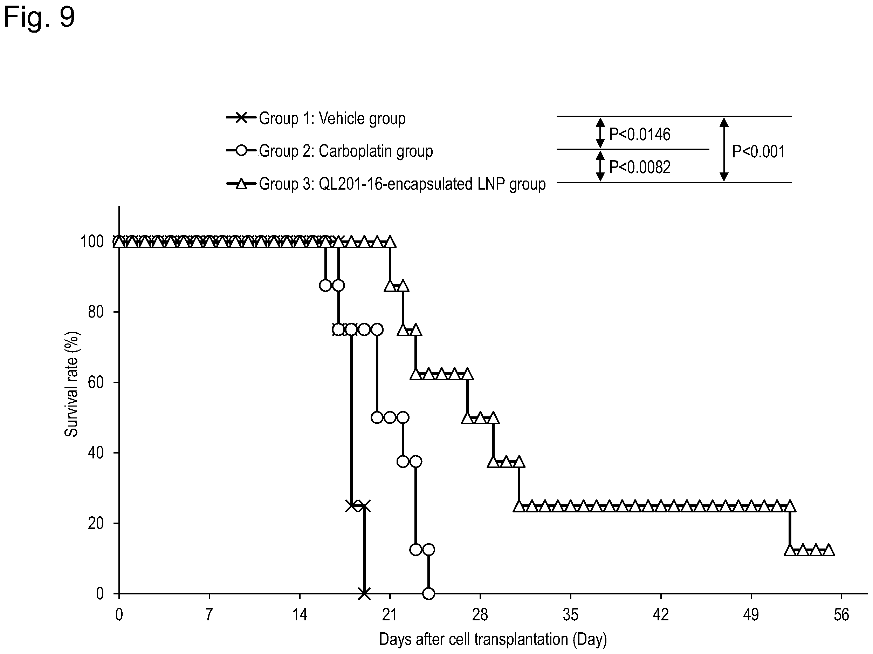

[Figure 9] Figure 9 is a graph showing the proportion of individuals surviving when saline, carboplatin, or QL201-16-encapsulated LNP were administered to model mice of peritoneal dissemination of human-derived ovarian cancer cell line ES-2. The horizontal axis in the figure indicates the day of cell transplantation, Day 0, and the p-value indicates a significant difference obtained using the log-rank test.

### Description of Embodiments

### <siRNA >

In one aspect of the present invention, an siRNA targeting a RecQL1 helicase gene, is provided.

Herein, the "siRNA" (short-interfering RNA) means a double-stranded nucleic acid having approximately 19 to 25 base pairs, which is capable of inducing reduction of the expression of a target gene via RNAi. An siRNA is composed of two nucleic acid strands, namely, the after-mentioned sense strand and antisense strand. The two nucleic acid strands constituting the siRNA of the present invention can comprise not only ribonucleosides but also deoxynucleosides and/or any modified nucleosides. This is because the siRNA capable of inducing RNAi activity is not limited to an siRNA consisting of a ribonucleoside, and an siRNA comprising a deoxynucleoside or a modified nucleoside can also be incorporated into RISC to be described later to be able to recognize a target mRNA. The siRNA may also comprise a single-stranded portion (overhang).

Herein, "RNAi" (RNA interference) means a phenomenon whereby the expression of a target gene is specifically reduced in cells, into which a double-stranded nucleic acid strand such as an siRNA comprising a sequence complementary to a sequence of a target gene has been introduced. The RNAi by the siRNA can be described as follows. First, one strand of the siRNA introduced into cells is incorporated into a complex called "RISC (RNA-induced Silencing Complex)," and recognizes the mRNA of a target gene having a highly complementary sequence. The mRNA of the target gene is cleaved at the central portion of the siRNA by RISC. Thereafter, the cleaved mRNA can be degraded.

The siRNA of the present invention targets a sequence comprising the sequence 5'-AGCAAUGAAUAUGAUUCUUCA-3' (SEQ ID NO: 18) of 21 bases in length at positions 683 to 703 in the mRNA sequence (SEQ ID NO: 17) of the human RecQL1 gene.

The siRNA of the present invention comprises: a sense strand comprising the base sequence of SEQ ID NO: 1, and an antisense strand comprising the base sequence of SEQ ID NO: 2. The base sequence of SEQ ID NO: 2 is a sequence complementary to the above-described base sequence (SEQ ID NO: 18) at positions 683 to 703 in an mRNA sequence of the human RecQL1 gene. The base sequence of SEQ ID NO: 1 corresponds to that at positions 685 to 705 in an mRNA sequence of a human RecQL1 gene. Herein, the term "base sequence of SEQ ID NO: 1" refers to including the sequence of the bases of SEQ ID NO: 1, regardless of the state of nucleoside modification at each position (presence or absence of modification at each position in the base sequence and the type of modification). The same applies to SEQ ID NO: 2. Therefore, it is understood that the "sense strand comprising the base sequence of SEQ ID NO: 1" includes both (i) a sense strand comprising the base sequence of SEQ ID NO: 1 and consisting of a natural ribonucleoside linked by a phosphodiester linkage and/or modified internucleoside linkage, or (ii) a sense strand comprising the base sequence of SEQ ID NO: 1 and comprising a natural deoxyribonucleoside or modified nucleoside, linked by a phosphodiester linkage and/or modified internucleoside linkage. It is also understood that the "antisense strand comprising the base sequence of SEQ ID NO: 2" comprises both (i) an antisense strand comprising the base sequence of SEQ ID NO: 2 and consisting of a natural ribonucleoside linked by a phosphodiester linkage and/or modified internucleoside linkage, or (ii) an antisense strand comprising the base sequence of SEQ ID NO: 2 and comprising a natural deoxyribonucleoside or modified nucleoside linked by a phosphodiester linkage and/or modified internucleoside linkage.

Herein, the "antisense strand" is used to mean a nucleic acid strand comprising a sequence complementary to mRNA of a target gene. Herein, the "sense strand" is used to mean a nucleic acid strand comprising a sequence complementary to the antisense strand (namely, comprising a sequence homologous to the mRNA of the target gene). The antisense strand is annealed to the sense strand to generate an siRNA. The antisense strand binds to the mRNA of a target gene, so that it can induce RNAi. Herein, the antisense strand constituting the siRNA binds to the positions 683 to 703 of RecQL1 mRNA, so that it can induce RNAi. Thus, the siRNA of the present invention can induce reduction of the expression of a RecQL1 gene.

The siRNA of the present invention may comprise a natural ribonucleoside, a natural deoxyribonucleoside, and/or a modified nucleoside.

Herein, the "natural nucleoside" is referred to as a nucleoside that is present in nature. Examples thereof include a ribonucleoside consisting of ribose and a base such as said adenine, cytosine, guanine, or uracil (referred to as a "natural ribonucleoside"), and a deoxyribonucleoside consisting of deoxyribose and a base such as said adenine, cytosine, guanine, or thymine (referred to as a "natural deoxyribonucleoside").

In one embodiment, the siRNA of the present invention is an unmodified siRNA. Herein, the "unmodified siRNA" means an siRNA composed of a deoxyribonucleoside and/or ribonucleoside having no modifications on sugar, bases, or phosphoric acids. In a case in which the siRNA of the present invention is the unmodified siRNA, the sense strand and the antisense strand thereof can consist of natural ribonucleosides and/or natural deoxyribonucleosides, linked by internucleoside linkages.

In one embodiment, the unmodified siRNA of the present invention consists of a sense strand comprising the base sequence of SEQ ID NO: 1 and consisting of a natural ribonucleoside linked by a phosphodiester linkage and an antisense strand comprising the base sequence of SEQ ID NO: 2 and consisting of a natural ribonucleoside linked by a phosphodiester linkage.

In one embodiment, the siRNA of the present invention is a modified siRNA. Herein, the "modified siRNA" means an siRNA comprising one or more modified nucleosides and/or modified internucleoside linkages.

Herein, the "modified nucleoside" means a nucleoside having modified sugar and/or a modified nucleobase.

Herein, the "modified sugar" refers to sugar having a substitution and/or any change from a natural sugar portion (i.e., a sugar portion found in DNA (2'-H) or RNA (2'-OH)). Herein, a nucleic acid strand may comprise one or more modified nucleosides, optionally comprising modified sugar. Herein, an example of the nucleoside having a modified sugar moiety includes, but is not limited to, a nucleoside comprising 2'-F (2'-fluoro group), 2'-OCH₃ (2'-OMe or 2'-O-methyl group), 2'-O(CH₂)₂OCH₃ (2'-O-MOE or 2'-O-methoxyethyl group), 5'-methyl (R or S), or 4'-S.

Herein, the "2'-modified sugar" means furanosyl sugar modified at the 2'-position. The substitution of the 2'-hydroxyl group in the 2'-modified sugar can be selected from a C₁-C₁₀ alkyl, an allyl, an amino, an azido, a thio, an -O- allyl, an -O- C₁-C₁₀ alkyl, -OCF₃, - O(CH₂)₂SCH₃, -O(CH₂)₂OCH₃, -O(CH₂)₂-O-N(Rm)(Rn), O-CH₂-C(=O)-N(Rm)(Rn), -O-(CH₂)₂-C(=O)-N(Rm)(Rn), and the like, and each Rm and Rn is independently H or a substituted or unsubstituted C₁-C₁₀ alkyl.

Herein, a nucleoside comprising 2'-modified sugar is referred to as a "2'-modified nucleoside" or a "2'-substituted nucleoside." The 2'-position of the 2'-modified nucleoside is - R¹, -OR¹, -R²OR¹, -OR²OR¹ or -R³OR²OR¹, wherein R¹ is a C₁₋₄ alkyl group, and R² and R³ are independently C₁₋₃ alkylene groups. Herein, the "alkyl group" means an optionally substituted, linear or branched, saturated or unsaturated, monovalent hydrocarbon group containing 1 to 4 carbon atoms. Examples of the "alkyl group" include a methyl group, an ethyl group, an n-propyl group, an isopropyl group, an n-butyl group, an isobutyl group, a sec-butyl group, and a tert-butyl group. Herein, the "alkylene group" means an optionally substituted, linear or branched, saturated or unsaturated, divalent hydrocarbon group containing 1 to 3 carbon atoms. Examples of the "alkylene group" include, for example, a methylene group, an ethylene group, and a trimethylene group. Examples of a substituent, which the alkyl group or the alkylene group may have, include, for example, a halogen atom (e.g., fluorine, chlorine, bromine, or iodine), an amino group, a nitro group, and a hydroxyl group. R¹ may be a C₁₋₃ alkyl group, a C₁₋₂ alkyl group, or a C₁ alkyl group. R² and R³ may each be a C₁₋₂ alkylene group or a C₁ alkylene group. Specific examples of the substituent at position 2' of the 2'-modified nucleoside include, for example, 2'-F(2'-fluoro group), -OCH₃ (methoxy), - OCH₂CH₃ (ethoxy), -OCH₂NH₂ (aminomethoxy), -OCH₂CH₂NH₂ (aminoethoxy), - OCH₂CH₂F (methyl methoxy fluoride), -OCH₂CH₂CH₂F (methyl ethoxy fluoride), -CH₃ (methyl), -CH₂CH₃ (ethyl), -CH₂CH₂CH₃ (propyl), -CH₂OCH₃ (methoxymethyl; MOM), - CH₂CH₂OCH₃ (methoxyethyl; MOE), -OCH₂OCH₃, -OCH₂CH₂OCH₃, -CH₂OCH₂OCH₃, and - CH₂OCH₂CH₂OCH₃ (methoxyethoxymethyl; MEM), but the examples are not limited thereto. The 2'-modified nucleoside is preferably a 2'-methoxy nucleoside (2'-O-methyl-modified nucleoside) or a 2'-fluoro-modified nucleoside. The sense strand and/or antisense strand, which constitute the siRNA of the present invention, can comprise a plurality of 2'-modified nucleosides as described above, however, the substituents at position 2' of the 2'-modified nucleosides may be identical to or different from one another.

In one embodiment, the modified nucleoside comprised in the siRNA of the present invention is a 2'-modified nucleoside and/or a bridged nucleoside.

Herein, the "bridged nucleoside" means a nucleoside comprising a bicyclic sugar moiety. A nucleic acid comprising the bicyclic sugar moiety is generally referred to as a bridged nucleic acid. The bicyclic sugar may be sugar in which the carbon atom at position 2' and the carbon atom at position 4' are bridged by two or more atoms. Examples of the bicyclic sugars are known to a person skilled in the art. Examples of nucleic acids (BNAs) comprising bicyclic sugar include, but are not limited to, a methyleneoxy (4'-CH₂-O-2') BNA (also known as "LNA"), an ethyleneoxy (4'-(CH₂)₂-O-2') BNA (also known as "ENA"), a cEt BNA, a cMOE BNA, an AmNA, a GuNA, and the like.

Herein, the "modified nucleobase" or "modified base" means any nucleobase other than adenine, cytosine, guanine, thymine, or uracil. Examples of the modified nucleobases include 5-methylcytosine, 5-fluorocytosine, 5-bromocytosine, 5-iodocytosine, N4-methylcytosine, N6-methyladenine, 8-bromoadenine, N2-methylguanine, or 8-bromoguanine, but are not limited thereto.

In one embodiment, all or part of the internucleoside linkages in the sense strand and/or antisense strand of the siRNA of the present invention are modified internucleoside linkages.

Herein, the "modified internucleoside linkage" means an internucleoside linkage having a substitution or any change from an internucleoside linkage that is present in nature (i.e., a phosphodiester linkage). The modified internucleoside linkage includes a phosphorus-containing internucleoside linkage comprising phosphorus atoms and a non-phosphorus-containing internucleoside linkage free of phosphorus atoms. Representative examples of the phosphorus-containing internucleoside linkages include a phosphorothioate linkage, a phosphorodithioate linkage, a phosphotriester linkage, an alkylphosphonate linkage, an alkylthiophosphonate linkage, a phosphorodiamidate, and the like, but are not limited to thereto. The phosphorothioate linkage is an internucleoside linkage in which a non-bridged oxygen atom of a phosphodiester linkage is replaced with a sulfur atom.

In another embodiment, all or part of the internucleoside linkages of the sense strand and/or antisense strand of the siRNA of the present invention are phosphodiester linkages.

In one embodiment, the sense strand and/or antisense strand of the siRNA of the present invention comprises a 2'-modified nucleoside or a bridged nucleoside at the 3' end. The 2'-modified nucleoside may be, for example, the 2'-O-methyl-modified nucleoside or the 2'-fluoro-modified nucleoside. The bridged nucleoside may also be, for example, an LNA nucleoside or an ENA nucleoside. Arrangement of 2'-modified nucleoside or bridged nucleoside at the 3' end allows stability of the siRNA of the present invention to be improved.

In one embodiment, in the sense strand of the siRNA of the present invention, the nucleoside at position 7 in the base sequence of SEQ ID NO: 1 is not the 2'-O-methyl-modified nucleoside. For example, the nucleoside at position 7 in the base sequence of SEQ ID NO: 1 may be a natural ribonucleoside, a natural deoxyribonucleoside, or a 2'-modified nucleoside other than the 2'-O-methyl-modified nucleoside (for example, the 2'-fluoro-modified nucleoside).

In one embodiment, the sense strand of the siRNA of the present invention comprises a 2'-modified nucleoside at one or more positions of positions 9 to 11 in the base sequence of SEQ ID NO: 1. This 2'-modified nucleoside may be the 2'-O-methyl-modified nucleoside or the 2'-fluoro-modified nucleoside.

In one embodiment, the sense strand of the siRNA of the present invention is a sense strand in which the nucleosides at position 9 and position 11 in the base sequence of SEQ ID NO: 1 are the same modified nucleoside except for the base moiety, or are the same natural nucleoside. Herein, the natural nucleoside may be a natural ribonucleoside or a natural deoxyribonucleoside. In a further embodiment, the sense strand of the siRNA of the present invention is a sense strand in which the nucleosides at position 9 to position 11 of the base sequence of SEQ ID NO: 1 are the same modified nucleoside except for the base moiety, or are the same natural nucleoside. Herein, the natural nucleoside may be a natural ribonucleoside or a natural deoxyribonucleoside.

In one embodiment, the sense strand of the siRNA of the present invention does not contain 2'-O-methyl-modified nucleosides at positions 7, 9, and/or 11 of the base sequence of SEQ ID NO: 1. For example, the sense strand comprises natural ribonucleosides, natural deoxyribonucleosides, and/or 2'-modified nucleosides other than the 2'-O-methyl-modified nucleoside (for example, the 2'-fluoro-modified nucleoside) at position 7, position 9, and/or position 11 of the base sequence of SEQ ID NO: 1.

In one embodiment, the sense strand of the siRNA of the present invention is a sense strand in which the nucleoside at position 10 of the base sequence of SEQ ID NO: 1 is not the 2'-O-methyl-modified nucleoside. For example, the nucleoside at position 10 of the base sequence of SEQ ID NO: 1 may be a natural ribonucleoside, a natural deoxyribonucleoside, or a 2'-modified nucleoside other than the 2'-O-methyl-modified nucleoside (for example, the 2'-fluoro-modified nucleoside). The nucleoside at position 10 in the base sequence of SEQ ID NO: 1, when it is not the 2'-O-methyl-modified nucleoside in the sense strand, enables the off-target effect caused by the siRNA to be reduced.

In one embodiment, the antisense strand of the siRNA of the present invention comprises 2'-modified nucleosides at positions 2 to 5 (preferably at position 2) of the base sequence of SEQ ID NO: 2. This 2'-modified nucleoside may be the 2'-O-methyl-modified nucleoside. The antisense strand of the siRNA comprising the 2'-O-methyl-modified nucleosides at positions 2 to 5 (preferably at position 2) of the base sequence of SEQ ID NO: 2 enables reducing the off-target effect caused by the siRNA.

In one embodiment, the antisense strand of the siRNA of the present invention does not contain 2'-O-methyl-modified nucleosides at position 6 and/or position 12 of the base sequence of SEQ ID NO: 2. For example, the antisense strand comprises natural ribonucleosides, natural deoxyribonucleosides, and/or 2'-modified nucleosides other than the 2'-O-methyl-modified nucleoside (for example, the 2'-fluoro-modified nucleoside) at position 6 and/or position 12 of the base sequence of SEQ ID NO: 2.

In one embodiment, the antisense strand of the siRNA of the present invention is an antisense strand in which the nucleoside at position 14 of the base sequence of SEQ ID NO: 2 is not the 2'-O-methyl-modified nucleoside. For example, the nucleoside at position 14 of the base sequence of SEQ ID NO: 2 may be a natural ribonucleoside, a natural deoxyribonucleoside, or the 2'-modified nucleoside other than the 2'-O-methyl-modified nucleoside (for example, the 2'-fluoro-modified nucleoside).

In one embodiment, the antisense strand of the siRNA of the present invention comprises 2'-modified nucleosides at one or more of positions 9 to 11 in the base sequence of SEQ ID NO: 2. This 2'-modified nucleoside may be the 2'-O-methyl-modified nucleoside or the 2'-fluoro-modified nucleoside.

In one embodiment, the antisense strand of the siRNA of the present invention is an antisense strand in which the nucleosides at position 9 and position 11 in the base sequence of SEQ ID NO:2 are the same modified nucleoside or are the same natural nucleoside except for the base portion. Herein, the natural nucleoside may be a natural ribonucleoside or a natural deoxyribonucleoside. In a further embodiment, the antisense strand of the siRNA of the present invention is an antisense strand in which the nucleosides at positions 9 to 11 in the base sequence of SEQ ID NO: 2 are the identical modified nucleoside or the identical natural nucleoside except for the base portion. Herein, the natural nucleoside may be a natural ribonucleoside or a natural deoxyribonucleoside.

In one embodiment, the antisense strand of the siRNA of the present invention is an antisense strand in which the nucleoside at position 10 in the base sequence of SEQ ID NO: 2 is not a 2'-O-methyl-modified nucleoside. For example, the nucleoside at position 10 in the base sequence of SEQ ID NO: 2 may be a natural ribonucleoside, a natural deoxyribonucleoside, or the 2'-modified nucleoside other than the 2'-O-methyl-modified nucleoside (for example, the 2'-fluoro-modified nucleoside). In the antisense strand, the nucleoside at position 10 in the base sequence of SEQ ID NO: 2, when it is not the 2'-O-methyl-modified nucleoside, can reduce the off-target effect caused by the siRNA.

In a further embodiment, the sense strand and the antisense strand of the siRNA of the present invention each comprises or consists of:
(a) the base sequence of SEQ ID NO: 3 and the base sequence of SEQ ID NO: 4,
(b) the base sequence of SEQ ID NO: 5 and the base sequence of SEQ ID NO: 6,
(c) the base sequence of SEQ ID NO: 7 and the base sequence of SEQ ID NO: 8,
(d) the base sequence of SEQ ID NO: 9 and the base sequence of SEQ ID NO: 10, or
(e) the base sequence of SEQ ID NO: 11 and the base sequence of SEQ ID NO: 12.
Herein, the "base sequence of SEQ ID NO: 3" means a nucleoside sequence comprising both the base sequence of SEQ ID NO: 3 and the state of nucleoside modification of SEQ ID NO: 3 (the presence or absence of modification at each position in the base sequence and the type of modification). The same applies to SEQ ID NOs: 4 to 12. All or part of the internucleoside linkages in the sense strand and/or antisense strand of the embodiments (a) to (e) may be modified internucleoside linkages (for example, phosphorothioate linkages) or phosphodiester linkages. The above-described embodiments (a) to (e) are shown in Table 1 below.

**[Table 1]**

| Table 1: Exemplified sense strands and antisense strands constituting siRNA | | | |
|---|---|---|---|
| | Nucleic acid strand | Sequence (5'-3') | SEQ ID NO |
| Embodiment (a) | Sense strand | caaugaauaugauucuucacc | 3 |
| | Antisense strand | ugaagaaucauauucauugcu | 4 |
| Embodiment (b) | Sense strand | caaugaAuAuGauucuucacc | 5 |
| | Antisense strand | ugaagAaucauauucauugcu | 6 |
| Embodiment (c) | Sense strand | caaugaAuAuGauucuucacc | 7 |
| | Antisense strand | ugaagAaucauauucauugcu | 8 |
| Embodiment (d) | Sense strand | cAAuGAAuAUGAuuCUUCAcc | 9 |
| | Antisense strand | UgaagAAucauAuucAuuGcu | 10 |
| Embodiment (e) | Sense strand | caaugaauaugauucuucacc | 11 |
| | Antisense strand | ugaagaaucauauucauugcu | 12 |

| | | | |
|---|---|---|---|
| Capital letter: RNA; Underlined capital letter: DNA; Lower case letter: 2'-O-methylated nucleoside; Underlined lower case letter: 2'-fluoro-modified nucleoside. All or part of the internucleoside linkages are modified internucleoside linkages (e.g. phosphorothioate linkages) or phosphodiester linkages. | | | |

It is generally known that, when an siRNA has a single-stranded portion (overhang) of several (for example, 2 to 5) nucleosides linked by internucleoside linkages at the terminal thereof, it has high RNAi activity. As such, the siRNA of the present invention may have an overhang consisting of several natural nucleosides or modified nucleosides, linked by internucleoside linkages at the terminal of the siRNA. In one embodiment, the siRNA of the present invention may have a 3'-overhang of two bases in length. For example, the siRNA of the present invention may have a 3'-overhang consisting of dithymidylic acid (TT) or diuridylic acid (UU).

The sense strand and the antisense strand, which constitute the siRNA of the present invention, may each be 21 to 25 bases in length. The sense strand and the antisense strand may have the same length as each other, or may have a different length from each other. That is to say, the sense strand may be composed of the base sequence of SEQ ID NO: 1 (21 bases in length), or may also have, for example, 1 to 4, 1 to 3, 1 or 2, or 1 natural nucleosides and/or modified nucleosides (*e.g*., base sequence homologous to RecQL1 mRNA, or UU or TT) at the 5'- and/or 3'-terminus thereof, in addition to the aforementioned sequence. Also, the antisense strand may be composed of the base sequence of SEQ ID NO: 2 (21 bases in length), or may also have, for example, 1 to 4, 1 to 3, 1 or 2, or 1 natural nucleosides and/or modified nucleosides (*e.g*., base sequence homologous to RecQL1 mRNA, or UU or TT) at the 5'-and/or 3'-terminus thereof, in addition to the aforementioned sequence. The sense strand and/or the antisense strand are preferably 21 to 23 bases in length, and more preferably 21 bases in length.

The present inventors had previously reported that mitotic catastrophe and mitotic cell death are induced in cancer cells by suppression of the expression of a RecQL1 gene mediated by an RNAi mechanism by an siRNA (Futami, K., et al. (2008) Cancer Sci., 99(1): 71-80; Futami, K., et al. (2008) Cancer Sci., 99(6): 1227-1236; and Futami, K., et al. (2010) Int. J. Mol. Med., 25: 537-545). The siRNA of the present invention targets the RecQL1 gene, and can induce cell death in cancer cells by suppression of the expression of the RecQL1 gene mediated by the RNAi mechanism. The siRNA of the present invention has significantly higher activity of reducing expression, reduced toxicity, and higher stability in blood, compared to conventional siRNAs such as QL46 and QL46-19 in the Examples described later.

The sense strand and the antisense strand, which constitute the siRNA of the present invention, can be produced by a common method that is well known in the art. The sense strand and the antisense strand can be produced, for example, by enzymatically or chemically synthesizing them in a manual or automatic reaction. When an RNA or DNA molecule is chemically synthesized, contract manufacturing service offered by manufacturers (*e.g*., GeneDesign, Inc., Dharmacon, QIAGEN, Sigma-Aldrich Co. LLC, etc.) may be used. In such a case, the types and positions of 2'-modified nucleoside(s) can be designated. The synthesized antisense strand and sense strand may be purified from a mixture, for example, according to extraction using a solvent or a resin, precipitation, electrophoresis, chromatography. The siRNA of the present invention can be produced by mixing the thus obtained sense strand and antisense strand, and annealing them to each other.

The siRNA of the present invention can be introduced into cells, tissues, or an individual body *in vitro* or *in vivo,* and it can be then used to induce inhibition of the expression of a target gene RecQL1 via RNAi. Moreover, when the siRNA of the present invention is introduced into cancer cells, it can induce cell death to the cancer cells by inhibition of the expression of the target gene RecQL1. Introduction of the siRNA can be appropriately carried out by a person skilled in the art according to a method known in the art. The siRNA may be introduced, for example, by physical methods such as direct injection of a solution containing the siRNA (for example, a microinjection method), bombardment using particles coated with the siRNA, or electroporation in the presence of the siRNA. Alternatively, the siRNA may be introduced by other methods for introducing a nucleic acid into cells, which are known in the art, such as lipid-mediated carrier transport (*e.g*., lipofection method using lipofectamine) or chemically mediated transport (*e.g*., gene transfer method using polyethyleneimine (PEI), DEAE-Dextran method, and calcium phosphate method), and further, the siRNA may also be introduced into cells by known drug delivery systems (DDS), such as liposome or polymeric micelles. Further examples of gene introduction methods (transformation methods) can refer to Green & Sambrook, 2012, Molecular Cloning: A Laboratory Manual Fourth Ed., Cold Spring Harbor Laboratory Press, Cold Spring Harbor, New York, etc.

Cells, tissues or individuals, into which the siRNA of the present invention is to be introduced, may be those derived from primates (*e.g*., a rhesus monkey, a cynomolgus monkey, a chimpanzee). Cells, tissues or individuals derived from humans are preferable.

The siRNA of the present invention is based on a novel target sequence in a RecQL1 gene and has improved RNAi activity against cancer cells, low toxicity to normal cells, and/or stability in blood, compared to those of conventional siRNAs.

Herein, the "toxicity" means an effect causing undesirable symptoms or functional abnormalities in a subject. The toxicity may be toxicity in any organ, such as hepatotoxicity, nephrotoxicity, or neurotoxicity. Herein, the toxicity also includes cytotoxicity and off-target toxicity (off-target effect).

### <Agent for reducing expression of RecQL1 gene, agent for inducing cell death, and agent for treating cancer>

In one aspect of the present invention, an agent for reducing the expression of a RecQL1 gene is provided containing the siRNA of the present invention as an active ingredient.

Herein, "reduction (suppression) of gene expression" means that the expression of the mRNA and/or protein of a target gene is reduced. The "reduction of gene expression" does not only mean that the expression of a gene is reduced by 100% relative to the case when an siRNA is not introduced, when the expression level of the gene is determined using the expression level of the mRNA or protein of the gene as an indicator, but it also means that the expression of the gene is reduced by 75% or more, 50% or more, 20% or more, or 10% or more. The degree of reduction of gene expression can be determined using the expression level of the mRNA or protein of the target gene as an indicator. The expression level of mRNA can be determined by *e.g*., Northern hybridization and quantitative RT-PCR, whereas the expression level of a protein can be determined by *e.g*., Western blotting, ELISA, measurement of protein activity, the fluorescent intensity of a fluorescent protein. Moreover, herein, the "RNAi activity" of the siRNA may be determined based on the expression level of a RecQL1 gene (the expression level of mRNA or a protein), or may also be determined based on the cell death-inducing activity of the siRNA to cancer cells. The above-described agent for reducing the expression of a RecQL1 gene expression may consist only of the siRNA of the present invention, or comprise other components such as pharmaceutically acceptable carriers or additives. The agent for reducing the expression of a RecQL1 gene expression may be used as a research reagent (RNAi reagent), or may also be used as a medicine in the treatment of diseases.

In one aspect of the present invention, an agent for inducing cell death or an agent for treating cancer, comprising the siRNA of the present invention, as an active ingredient, is provided. The agent for inducing cell death or the agent for treating cancer of the present invention does not substantially induce cell death to normal cells, but can effectively induce cell death to cancer cells. The above-described agent for inducing cell death or agent for treating cancer may consist only of the siRNA of the present invention or may comprise other components such as pharmaceutically acceptable carriers or additives. The agent for inducing cell death may be used as a research reagent, or may also be used as a medicine in the treatment of diseases.

### <Pharmaceutical composition for treating cancer>

In one aspect of the present invention, a pharmaceutical composition for treating cancer is provided, comprising the siRNA of the present invention.

Herein, the type of "cancer" is not limited, and examples thereof include adenocarcinoma, squamous cell carcinoma, small cell carcinoma, large cell carcinoma, etc. Specific examples of cancer types include, for example, malignant melanoma (melanoma), skin cancer, oral cancer, laryngeal cancer, pharyngeal cancer, thyroid cancer, lung cancer, breast cancer, esophageal cancer, gastric cancer, colorectal cancer (including colon cancer and rectal cancer), small intestine cancer, pancreatic cancer, bladder cancer, prostate cancer, testicular cancer, uterine cancer, cervical cancer, endometrial cancer, ovarian cancer, kidney cancer, liver cancer, pancreatic cancer, biliary tract cancer (including gallbladder cancer and bile duct cancer), brain tumor, head and neck cancer, mesothelioma, osteosarcoma, soft tissue sarcoma, glioma, pediatric tumors such as neuroblastoma, blood cancer, lymphoma, and myeloma. Examples of blood cancers include leukemias (*e.g*., B-cell leukemia, acute lymphoblastic leukemia, chronic lymphocytic leukemia), lymphoma (*e.g*., non-Hodgkin's lymphoma), and myelomas (*e.g*., multiple myeloma). Also, ovarian cancer is generally classified into endometrioid adenocarcinoma, serous adenocarcinoma, clear cell adenocarcinoma, and mucinous adenocarcinoma. The cancer to be treated is preferably ovarian cancer, and more preferably ovarian clear cell adenocarcinoma. In one embodiment, the cancer to be treated by the pharmaceutical composition for treating cancer of the present invention is ovarian cancer, breast cancer, malignant melanoma (melanoma), gastric cancer, pancreatic cancer, liver cancer, colorectal cancer, lung cancer, head and neck cancer, peritoneal cancer, or cervical cancer. Gastric cancer, pancreatic cancer, colorectal cancer, and ovarian cancer are known to cause peritoneal dissemination.

The pharmaceutical composition of the present invention can further comprise pharmaceutically acceptable carriers, as necessary. The pharmaceutically acceptable carriers include a diluent and an excipient, and specific examples of the pharmaceutically acceptable carriers include maltose, mannitol, lactose, xylose, trehalose, sorbitol, gelatin, gum Arabic, guar gum, tragacanth, ethanol, normal saline, Ringer's solution, etc.

The pharmaceutical composition of the present invention may also comprise additives such as a stabilizer, a buffer agent, an emulsifier, a tonicity agent, and a preservative, as necessary, as well as the above-described carriers. These additives are preferably additives used upon drug manufacturing.

Examples of the stabilizers include, for example, albumin, gelatin, mannitol, and sodium EDTA. Examples of the buffer agents include sodium citrate, citric acid, sodium phosphate, etc. Examples of the emulsifiers include, for example, sorbitan fatty acid ester and glycerin fatty acid ester. Examples of the tonicity agents include, for example, sodium chloride, potassium chloride, and sugars. Examples of the preservative include, for example, benzalkonium chloride, parahydroxybenzoic acid, and chlorobutanol.

The pharmaceutical composition of the present invention can comprise other drugs, as long as the siRNA of the present invention comprised as an active ingredient does not lose its RNAi activity. For example, in the case of an injection, the pharmaceutical composition may comprise a certain amount of antibiotic.

Examples of the dosage form of the pharmaceutical composition include, but are not limited to, parenteral agents, such as an injection, an aerosolized agent, a mist, aerosol, eye drops, a cream agent, nasal drops, nasal spray, gels, an ointment, a transmucosal agent, plasters, and a suppository, and oral agents, such as a liquid agent, a powder agent, a tablet, a granule, suspensions, pills, powdered medicine, a capsule, a sublingual tablet, and lozenges. For example, when head and neck cancer or lung cancer is targeted, the aerosolized agent can be suitably used.

The pharmaceutical composition of the present invention can be administered to a subject or an object in a therapeutically effective amount for the treatment of disease of interest (cancer). Herein, the "therapeutically effective amount" means a dose necessary for the siRNA comprised in the pharmaceutical composition of the present invention to treat cancer as a target, or to alleviate symptoms, wherein the dose causes almost no or completely no side effects that are harmful for a living body, to which the pharmaceutical composition is administered. The specific dose is determined depending on each subject, for example, by a physician's discretion, based on *e.g*., the progression or severity of disease, the healthy conditions of a whole body, age, body weight, sex, tolerability to the treatment.

Herein, the "subject" means an object to which the siRNA, agent for treating cancer, or pharmaceutical composition for treating cancer, of the present invention is applied. The subject includes individuals as well as organs, tissues, and cells. When the subject is an individual, it may be any animal, including a human. Examples of subjects other than humans include various livestock, poultry, pets, and laboratory animals. Although not limited thereto, the subject may be a cancer patient.

The administration of the pharmaceutical composition of the present invention may be either systemic administration or local administration (e.g., direct administration to affected area). The administration route may be either parenteral or oral administration. Examples of the administration route include, for example, intraperitoneal, intravenous, intraarterial, intrahepatic, intravaginal, intramuscular, intramedullary, intraspinal, transdermal, subcutaneous, intradermal, intranasal, intraoral, intrapharyngeal, pulmonary (*e.g*., inhalation through a mouth or nose), transrectal, intraintestinal, intrabronchial, intrapulmonary, or sublingual administrations.

When the pharmaceutical composition is applied by administration or ingestion, the dose or the amount ingested may be, for example, such that the amount of the siRNA contained therein is 0.001 mg/kg/day to 100 mg/kg/day. The dose of the above-described siRNA at once can be, for example, 0.001 mg/kg or more, 0.005 mg/kg or more, 0.01 mg/kg or more, 0.1 mg/kg or more, 0.25 mg/kg or more, 0.5 mg/kg or more, 1.0 mg/kg or more, 2.0 mg/kg or more, 2.5 mg/kg or more, 3.0 mg/kg or more, 4.0 mg/kg or more, 5 mg/kg or more, 10 mg/kg or more, 20 mg/kg or more, 30 mg/kg or more, 40 mg/kg or more, 50 mg/kg or more, 75 mg/kg or more, 100 mg/kg or more, 150 mg/kg or more, 200 mg/kg or more, 300 mg/kg or more, 400 mg/kg or more, or 500 mg/kg or more, and, for example, any amount within the range of 0.001 mg/kg to 500 mg/kg (*e.g*., 0.001 mg/kg, 0.01 mg/kg, 0.1 mg/kg, 1 mg/kg, 5 mg/kg, 10 mg/kg, 50 mg/kg, 100 mg/kg, or 200 mg/kg) can be appropriately selected.

Moreover, the pharmaceutical composition of the present invention can be administered to a patient, for example, based on a therapeutic plan determined by a physician, at certain time intervals, for example, at intervals of 1 day, 2 days, 3 days, 4 days, 5 days, 6 days, 1 week, 2 weeks, 3 weeks, 1 month, 2 months, 6 months, 1 year, or the like, at once, or divided over several to several tens of administrations.

There is also provided a method for treating cancer, or a method for inducing cell death of cancer cells in a cancer patient, comprising the step of administering the siRNA, agent for treating cancer, or pharmaceutical composition for treating cancer, of the present invention to a subject.

Also provided is use of the siRNA of the present invention in the manufacture of medicine for treating cancer.

### Examples

Hereinafter, the present invention will be further specifically described in the following examples. However, these examples are not intended to limit the technical scope of the present invention.

### <Example 1: Preparation of siRNA targeting RecQL1 helicase gene>

### (Objective)

An siRNA targeting the RecQL1 helicase gene is prepared.

### (Method and results)

The constitution of the siRNA prepared in the present Examples is shown in Table 2 and Figure 1 below.

**[Table 2]**

| Table 2: siRNA targeting RecQL1 helicase gene | | | |
|---|---|---|---|
| | Strand | Sequence (5'-3') | SEQ ID NO |
| QL46 | S strand | GUUCAGACCACUUCAGCUUTT | 13 |
| | AS strand | AAGCUGAAGUGGUCUGAACTT | 14 |
| QL46-19 | S strand | GuucAGACCACuucAGcuuTT | 15 |
| | AS strand | AAGCUGAAGUGGuCuGAAcTT | 16 |
| QL201 | S strand | CAAUGAAUAUGAUUCUUCACC | 1 |
| | AS strand | UGAAGAAUCAUAUUCAUUGCU | 2 |
| QL201-16 | S strand | caaugaauaugauucuucacc | 3 |
| | AS strand | ugaagaaucauauucauugcu | 4 |
| QL201-29 | S strand | caaugaAuAuGauucuucacc | 5 |
| | AS strand | ugaagAaucauauucauugcu | 6 |
| QL201-30 | S strand | caaugaAuAuGauucuucacc | 7 |
| | AS strand | ugaagAaucauauucauugcu | 8 |
| QL201-13 | S strand | cAAuGAAuAUGAuuCUUCAcc | 9 |
| | AS strand | UgaagAAucauAuucAuuGcu | 10 |
| QL201-15 | S strand | caaugaauaugauucuucacc | 11 |
| | AS strand | ugaagaaucauauucauugcu | 12 |

| | | | |
|---|---|---|---|
| Capital letter: RNA; Underlined capital letter: DNA; Lower case letter: 2'-O-methylated nucleoside; Underlined lower case letter: 2'-fluoro-modified nucleoside. All of the internucleoside linkages are phosphorothioate linkages. In the table, the "S strand" means a sense strand, and the "AS strand" means an antisense strand. | | | |

The siRNA prepared in the present Examples is an siRNA targeting the RecQL1 helicase gene, and comprises a base sequence complementary to a part of the mRNA of the human RecQL1 helicase gene as an antisense strand. QL46 corresponds to RERQ1-1 (SEQ ID NO: 1 in the following international publication) described in International Publication WO 2004/100990 and the "unmodified siRNA" described in International Publication WO 2017/022650, and QL46-19 corresponds to "QL-19" in International Publication WO 2017/022650. Both of them are conventional siRNAs and are used for the purpose of comparison with the siRNA of the present invention. QL201, QL201-16, QL201-29, QL201-30, QL201-13, and QL201-15 are siRNAs newly prepared herein. The specific constitution of each siRNA is as follows.

QL46 comprises a sense strand consisting of the base sequence of SEQ ID NO: 13 and an antisense strand consisting of the base sequence of SEQ ID NO: 14. The sense strand of QL46 comprises a base sequence corresponding to that at positions 784 to 802 of the mRNA of the human RecQL1 helicase gene (Homo sapiens RecQ like helicase (RECQL), transcript variant 1, mRNA; NCBI accession number NM_002907.4; SEQ ID NO: 17), and has a structure in which each of 19 natural ribonucleosides on the 5'-terminus and two natural deoxyribonucleosides on the 3'-terminus, are linked by phosphodiester linkages. The antisense strand of QL46 comprises a base sequence complementary to the sequence at positions 784 to 802 of the above mRNA, and has a structure in which each of 19 natural ribonucleosides on the 5'-terminus and two natural deoxyribonucleosides on the 3'-terminus, are linked by phosphodiester linkages.

QL46-19 comprises a sense strand consisting of the base sequence of SEQ ID NO: 15, and an antisense strand consisting of the base sequence of SEQ ID NO: 16. The sense strand of QL46-19 comprises 2'-O-methyl-modified nucleosides at positions 2 to 4, 12 to 14, and 17 to 19 in the sense strand of QL46. The antisense strand of QL46-19 comprises 2'-O-methyl-modified nucleosides at positions 13, 15, and 19 in the antisense strand of QL46.

QL201 comprises a sense strand consisting of the base sequence of SEQ ID NO: 1, and an antisense strand consisting of the base sequence of SEQ ID NO:2. The sense strand of QL201 comprises a base sequence corresponding to that at positions 685 to 705 in the mRNA of the human RecQL1 helicase gene, and has a structure in which natural ribonucleosides are linked by phosphodiester linkages. The antisense strand of QL201 comprises a base sequence complementary to that at positions 683 to 703 of the above mRNA, and has a structure in which natural ribonucleosides are linked by phosphodiester linkages.

QL201-16 comprises a sense strand consisting of the base sequence of SEQ ID NO:3, and an antisense strand consisting of the base sequence of SEQ ID NO:4. The sense strand of QL201-16 has 2'-O-methyl-modified nucleosides at positions 1 to 6, 8, and 12 to 21 and 2'-fluoro-modified nucleosides at positions 7 and 9 to 11 in the sense strand of QL 201. The antisense strand of QL201-16 has 2'-O-methyl-modified nucleosides at positions 1, 3 to 5, 7 to 11, 13, and 15 to 21 and 2'-fluoro modified nucleosides at positions 2, 6, 12, and 14 in the antisense strand of QL201.

QL201-29 comprises a sense strand consisting of the base sequence of SEQ ID NO: 5, and an antisense strand consisting of the base sequence of SEQ ID NO: 6. The sense strand of QL201-29 has 2'-O-methyl modified nucleosides at positions 1 to 6, 8, 9, 10, and 12 to 21 and natural ribonucleoside at positions 7, 9 and 11 in the sense strand of QL201. The antisense strand of QL201-29 has 2'-O-methyl-modified nucleosides at positions 1, 3 to 5, 7 to 9, 11, 13, and 15 to 21, 2'-fluoro-modified nucleosides at positions 2, 10, 12, and 14, and a natural ribonucleoside at position 6 in the antisense strand of QL201.

QL201-30 comprises a sense strand consisting of the base sequence of SEQ ID NO: 7, and an antisense strand consisting of the base sequence of SEQ ID NO: 8. The sense strand of QL201-30 has 2'-O-methyl-modified nucleosides at positions 1 to 6, 8, 10, and 12 to 21 and natural ribonucleosides at positions 7, 9, and 11 in the sense strand of QL201. The antisense strand of QL201-30 has 2'-O-methyl-modified nucleosides at positions 1, 3 to 5, 7 to 11, 13, and 15 to 21, 2'-fluoro-modified nucleosides at positions 2, 12, and 14, and a natural ribonucleoside at position 6 in the antisense strand of QL201.

QL201-13 comprises a sense strand consisting of the base sequence of SEQ ID NO: 9, and an antisense strand consisting of the base sequence of SEQ ID NO: 10. The sense strand of QL201-13 has 2'-O-methyl-modified nucleosides at positions 1, 4, 8, 10, 13, 14, 20, and 21 and natural ribonucleosides at positions 2, 3, 5 to 7, 9 to 12, and 15 to 19 in the sense strand of QL201. The antisense strand of QL201-13 has 2'-O-methyl-modified nucleosides at positions 2 to 5, 8 to 11, 13 to 15, 17, 18, 20, and 21 and natural ribonucleosides at positions 1, 6, 7, 12, 16, and 19 in the antisense strand of QL201.

QL201-15 comprises a sense strand consisting of the base sequence of SEQ ID NO: 11, and an antisense strand consisting of the base sequence of SEQ ID NO: 12. The sense strand of QL201-15 has 2'-O-methyl-modified nucleosides at positions 2, 4, 6, 8, 12, 14, 16, 18, and 20 and 2'-fluoro-modified nucleosides at positions 1, 3, 5, 7, 9 to 11, 13, 15, 17, 19, and 21 in the sense strand of QL201. The antisense strand of QL201-15 has 2'-O-methyl-modified nucleosides at position 1, 3, 5, 7, 9 to 11, 13, 15, 17, and 19 to 21 and 2'-fluoro-modified nucleosides at positions 2, 4, 6, 8, 12, 14, 16, and 18 in the antisense strand of QL201.

The siRNAs described above underwent contract synthesis, purification, and annealing by Gene Design, Inc., which was then dissolved in Nuclease Free Water before use, and used.

### <Example 2: Evaluation of RNAi activity to RecQL1 helicase gene>

### (Objective)

For identifying a target sequence of a new siRNA exhibiting superior RNAi activity to conventional siRNAs, the siRNA prepared in Example 1 was introduced into various cancer cell lines, and the effect on the expression level of the mRNA of the RecQL1 helicase gene is investigated.

It is noted that QL46, which is compared in the following examples, corresponds to the "unmodified siRNA" described in International Publication WO 2017/022650, and QL46-19 corresponds to "QL-19" in said international publication (collectively referred to as "conventional siRNAs" in the following examples).

### (Method)

### (1) Culture of cancer cell lines

The following human cell lines were used: ES-2 cells (ovarian cancer), HeLa cells (cervical cancer), A549 cells (lung cancer), Lovo cells (colorectal cancer), MKN45 cells (gastric cancer), and Panc-1 cells (pancreatic cancer). The cell lines, ES-2 cells (ATCC, product number CRL-1978), HeLa cells (ATCC, product number CCL-2.2), and Panc-1 cells (ATCC, product number CRL-1469) were obtained from ATCC (American Type Culture Collection), A549 cells (RIKEN (the Institute of Physical and Chemical Research), product number RCB0098) and LOVO cells (RIKEN, product number RCB 1639) were obtained from RIKEN, and MKN45 cells (JCRB, product number JCRB0254) were obtained from JCRB.

One day before siRNA transfection, the cells were plated at a cell density of 2.5 × 10⁴ cells per well to 4 × 10⁴ cells per well on a 24-well plate, and the cells were cultured. ES-2 cells were cultured using McCoy5A (GibcoBRL) + 10% NBCS (Newborn Calf Serum, Gibco). HeLa cells were cultured using DMEM (high glucose, Nacalai Tesque, Inc.) + 10% FBS (Fetal Bovine Serum, Gibco). A549 cells were cultured using EMEM (Wako Pure Chemical Industries, Ltd.) + 10% FBS + NEAA (non-Essential Amino Acids, Nacalai Tesque, Inc.). Lovo cells were cultured using EMEM (Wako Pure Chemical Industries, Ltd.) + 10% FBS. MKN45 cells were cultured using RPMI1640 (Nacalai Tesque, Inc.) + 10% FBS. Panc-1 cells were cultured using DMEM (high glucose, Nacalai Tesque, Inc.) + 20% FBS.

### (2) Introduction of siRNA

siRNAs at the concentrations described in each of the following examples were introduced into cells, using Lipofectamine^{™} RNAi MAX Reagent (Thermo Fisher Scientific) in accordance with protocols provided by the manufacturer.

It is also noted that in the following examples, cells in which only Lipofectamine^{™} RNAi MAX Reagent (Thermo Fisher Scientific) was added and the siRNA was not introduced, were used as a control group (Control).

### (3) Evaluation of the activity of reducing gene expression

Twenty-four hours after siRNA introduction, the cells were collected, and total RNA was extracted using NucleoZOL (MACHERY-NAGEL). The amount of RecQL1 mRNA (RecQL1 gene expression level) in the extracted RNA was determined by quantitative RT-PCR. Quantitative RT-PCR was carried out using the Rotor-Gene Q 2plex System (QIAGEN). RT-PCR primers and TaqMan probes for RecQL1 and β-actin genes were purchased from Applied Biosystems Inc. RT-PCR reactions were carried out using QuantiFast Probe RT-PCR Kit (QIAGEN GmbH) in accordance with protocols provided by the manufacturer.

The RecQL1 gene expression level was normalized to the β-actin gene expression level, and the relative expression level was then calculated assuming that the expression level of the control group (Control) is 1. Experiments for each siRNA were performed with n = 2 or 3, and the control group (Control) was performed with n = 4 to 6.

### (Results)

### (1) RNAi activity of unmodified siRNA targeting a novel sequence

A new target sequence of an siRNA exhibiting a superior RNAi effect compared to those of conventional siRNAs has been investigated. Specifically, each unmodified siRNA was introduced into ES-2 cells at concentrations of 0.0005 nM, 0.005 nM, 0.05 nM, 0.5 nM, and 5 nM, and a new target sequence of an siRNA capable of inducing a stronger RNAi effect than that of QL46, which was a conventional unmodified siRNA, was investigated. As a result, QL201 was found as an siRNA based on a new target sequence capable of inducing an overwhelmingly higher RNAi effect than that of QL46.

QL46 or QL201 was introduced into ES-2 cells at each concentration, and the measurement results of the relative expression level of the RecQL1 gene are shown in Figure 2. The introduction of QL201 significantly reduced the relative expression level of the RecQL1 gene. QL201 exhibited a significant gene expression-inhibiting effect compared to QL46. The difference between the two was particularly large when used at low concentrations (0.0005 nM to 0.05 nM). The above results demonstrated that QL201, which was based on the new target sequence, exhibits surprising efficacy compared to the conventional sequences.

### (2) RNAi activity of modified siRNA

QL201-16, QL201-29, QL201-30, QL201-13, and QL201-15, obtained by introduction of modified nucleosides into QL201 found in (1) above, were introduced into HeLa cells, and the effect on the expression level of the RecQL1 gene was investigated.

An siRNA of 0.005 nM was introduced into HeLa cells, and the measurement results of the relative expression level of the RecQL1 gene are shown in Figure 3. QL201-16, QL201-29, QL201-30, QL201-13, and QL201-15, which were the modified siRNAs, exhibited comparable gene expression-reducing effect as QL201, which was the unmodified siRNA. The effect was significantly higher than that of QL46, which was a conventional unmodified siRNA, and QL46-19, which was a conventional modified siRNA.

Next, siRNAs of 0.05 nM to 5 nM were introduced into ES-2 cells, and the measurement results of the relative expression level of the RecQL1 gene are shown in Figure 4. QL201-13 and QL201-15, which were the modified siRNAs, exhibited comparable gene expression-reducing effect as QL201, which was the unmodified siRNA.

### (3) RNAi activity in various cancer cell lines

Each siRNA (QL201, QL201-16, QL201-29, and QL201-30), having a concentration of 0.05 nM to 5 nM was introduced into each cell line of A549 cell lines, Lovo cell lines, MKN45 cell lines, and Panc-1 cell lines, and the measurement results of the relative expression level of the RecQL1 gene are shown in Figure 5. Each siRNA effectively reduced the gene expression in all the cancer cell lines.

All those results described above demonstrated that the unmodified QL201 and modified siRNA based on QL201 exhibited significant RNAi activity for the RecQL1 gene in the cancer cells derived from various cancer types.

### <Example 3: Investigation of toxicity to normal cells>

### (Objective)

The toxicity of QL201 and the modified siRNA based on QL201 to normal cells is examined. Specifically, immune responsiveness induced upon introduction of siRNA into normal cells was compared with that of a conventional siRNA. Specifically, the siRNA was introduced into a TIG-3 cell line (human-derived normal fibroblasts) and changes in the expression levels of an IFN-β gene, OAS-1 gene, and OAS-2 gene, are investigated.

### (Method)

In order to examine the toxicity to normal cells, the TIG-3 cell line (JCRB, product name TIG-3-20, product number JCRB0506) was used in the present Examples.

One day before siRNA transfection, the cells were plated at a cell density of 2 × 10⁴ cells per well on a 24-well plate, and the cells were then cultured. EMEM (Wako Pure Chemical Industries, Ltd.) + 10% FBS + NEAA (non-essential amino acids, Nacalai Tesque, Inc.) were used for the culture. After having replaced the medium with a new medium, siRNA having a concentration of 5 nM or 50 nM was introduced into the cells using Lipofectamine^{™} RNAi MAX Reagent (Thermo Fisher Scientific Inc.) in accordance with protocols provided by the manufacturer.

In the present Examples, cells in which the siRNA was not introduced and only Lipofectamine^{™} RNAi MAX Reagent (Thermo Fisher Scientific Inc.) was added were used as the control group. Also, cells to which 0.5 ng of Poly(I: C) was introduced were also used as a control.

Forty-eight hours after siRNA introduction, the cells were collected, and total RNA was then extracted using NucleoZOL (MACHERY-NAGEL). The expression levels of an IFN-β gene, OAS-1 gene, and OAS-2 gene in the extracted RNA were determined by quantitative RT-PCR. Quantitative RT-PCR was carried out using RNA-direct SYBR Green Realtime PCR Master Mix (TOYOBO CO., LTD., QRT-201).

The expression levels of the IFN-β gene, OAS-1 gene, and OAS-2 gene were normalized with respect to the expression level of an hTubulinB gene, and the relative expression levels were then calculated assuming that the expression level of the control group (Control) is 1. Experiments for each group were carried out with n = 3.

### (Results)

The measurement results of the expression levels of the IFN-β gene, OAS-1 gene, and OAS-2 gene are shown in Figure 6. It was found that QL201, QL201-16, QL201-29, and QL201-30 had lower immune responsiveness than QL46, which was a conventional unmodified siRNA, and QL46-19, which was a conventional modified siRNA. These results demonstrated that QL201, QL201-16, QL201-29, and QL201-30 had reduced toxicity compared to the conventional siRNAs.

### <Example 4: Investigation of stability of siRNA in blood>

### (Objective)

For the siRNA prepared in Example 1, stability thereof in human serum was evaluated.

### (Method and results)

Each siRNA (20 µM) of QL46, QL46-19, QL201, QL201-16, QL201-29, and QL201-30 in 10 µL was mixed with 10 µL of human serum and 80 µL of PBS, and incubated in a CO₂ incubator at 37°C. After 0 hour to 3 days, 20 µL of each sample was collected over time, mixed with 10 µL of 0.1 M EDTA, and then temporarily stored at -20°C. To 10 µL of the collected sample was added 2 µL of 6 × Loading Buffer, and the mixture was applied to 20% acrylamide gel (Native PAGE) and electrophoresed at 100V for 3 hours. The gel was stained with ethylene bromide, and the amount of the remaining siRNA was examined by the electrophoresis.

The electrophoresis results after incubation in human serum are shown in Figure 7.

QL46, which was a conventional unmodified siRNA, was completely degraded, with no bands observed after 2 hours of incubation. QL46-19, which was the conventional modified siRNA, was almost completely degraded, with no bands observed by 24 hours (Figure 7A).

On the other hand, the bands of QL201-16, QL201-29, and QL201-30, which were the modified siRNAs of the present invention, did not disappear for at least three days. This result demonstrated that the modified siRNAs of the present invention were stable in serum (Figure 7B).

All those results described above demonstrated that the stability of the modified siRNAs of the present invention in human serum was significantly increased compared to that of the conventional modified siRNAs.

### <Example 5: Evaluation of cell death-inducing activity>

### (Objective)

The cell death-inducing activity of QL201 prepared in Example 1 to ovarian cancer-derived ES-2 cells is evaluated.

### (Method and results)

ES-2 cells (ATCC, product number CRL-1978) were cultured in McCoy5A (GibcoBRL) + 10% NBCS (Newborn Calf Serum, Gibco) in the same manner as in Example 2. QL201, which was the unmodified siRNA of the present invention prepared in Example 1 (0.5 nM, 5 nM, or 50 nM), was introduced into the cells using Lipofectamine^{™} RNAi MAX Reagent (Thermo Fisher Scientific Inc.) in accordance with protocols provided by the manufacturer. It is noted that cells to which siRNA was not introduced and only Lipofectamine^{™} RNAi MAX Reagent (Thermo Fisher Scientific Inc.) was added were used as the control group. After siRNA introduction, the cells were cultured at 37°C for 120 hours in a CO₂ incubator. Using Celltiter Glo (registered trademark) 2.0 Assay (Promega, product number G9242), the luminescence intensity of each group (n = 6) was measured, and the survival rate was evaluated by calculating a relative value assuming that a survival rate of the control group is 100%.

The results are shown in Figure 8. The introduction of the unmodified siRNA of the present invention was found to be able to efficiently induce cell death in ES-2 cells.

### <Example 6: Survival rate evaluation test using mouse model of peritoneal dissemination of human-derived ovarian cancer cell line ES-2>

### (Objective)

QL201-16, which is the siRNA prepared in Example 1, is encapsulated in lipid nanoparticles (LNPs) and administered to a mouse model of peritoneal dissemination of human ovarian cancer cell line ES-2, and the survival rate after administration is evaluated.

### (Method and results)

### (1) Preparation of QL201-16-encapsulated lipid nanoparticles

Each of the following four types of lipids: CLZ-42 (HFX PHARMA, K.K), DSPC (Nippon Fine Chemical Co., Ltd., A90195), Cholesterol (hereafter abbreviated as "Cho") (Nippon Fine Chemical Co., Ltd., B71155), and DMG-MPEG2000 (Nippon Fine Chemical Co., Ltd., RHF-MD051) was dissolved in ethanol and mixed in a weight ratio of CLZ-42: DSPC: Cho: DMG-MPEG2k = 54:14:26:7 to prepare a lipid solution containing 32 mg/mL of lipid. Also, QL201-16, which was the siRNA prepared in Example 1, 100 mM of citrate buffer, and RNase free water were mixed to prepare a siRNA solution containing 0.68 mg/mL of QL201-16 and 1 mM of citrate buffer (pH 4.0).

Next, the above lipid solution and the siRNA solution were mixed at the ratio of 1: 3.58 and then dialyzed using PBS(-). The sample was recovered from a dialysis membrane, and the lipid nanoparticle (LNP) solution obtained after dialysis was adjusted to an siRNA concentration of 1 mg/mL using PBS(-), and then underwent sterile filtering using a 0.2 µm filter. In the subsequent examples, the sample after sterile filtration was used as QL201-16-encapsulated lipid nanoparticles (or QL201-16-encapsulated LNP) for administration.

### (2) Survival rate evaluation test

Human ovarian cancer cell line ES-2 was cultured in a medium (McCoy's 5A (Modified) Medium + 10% FBS + 1% Penicillin-Streptomycin Solution) and suspended in PBS (-) to obtain a cell suspension, which was then transplanted into abdominal cavities of 5-week-old female nude mice (CAnN.Cg-Foxn1nu/CrlCrlj; purchased from Jackson Laboratory Japan, Inc.). The number of transplanted cells was 2.0 × 10⁶ cells/mouse, and 0.2 mL of the cell suspension was transplanted into the abdominal cavity of each mouse.

The day of cell transplantation was designated Day 0, and for each administration group, test substances were administered to model mice of peritoneal dissemination of human ovarian cancer cell line ES-2, according to the following method. It is noted that eight mice were used for each group.

Group 1 (Vehicle group): As a negative control, saline was administered intraperitoneally (ip) at a dosage volume of 10 mL/kg. The administration was carried out once a day from Day 3 to Day 12, a total of 10 times.

Group 2 (Carboplatin group): As a positive control, carboplatin (75 mg/10 mL/kg) was administered intravenously into the tail vein (iv) on Days 3, 10, and 17, a total of 3 times.

Group 3 (QL201-16-encapsulated LNP group): The QL201-16-encapsulated lipid nanoparticles (QL201-16-encapsulated LNP, 2 mg/10 mL/kg) were administered intraperitoneally (ip) once a day from Day 3 to Day 12, a total of 10 times.

The day of cell transplantation was designated as Day 0, and the measurement results of the number of proportion of individuals surviving up to Day 56 are shown in Figure 9. The average survival time of each group was 18.0 days for Group 1 (Vehicle group), 20.6 days for Group 2 (Carboplatin group), and 32.6 days for gr Group oup 3 (QL201-16-encapsulated LNP group). The survival time of Group 3 (QL201-16-encapsulated LNP group) was significantly longer than that of Group 1 (Vehicle group) and Group 2 (Carboplatin group) (p values in the figure are those obtained by a log-rank test), indicating that QL201-16, which was the siRNA of the present invention had an excellent therapeutic effect on tumors.

The above-described all those results demonstrated that the siRNA of the present invention exhibited effective therapeutic effects on tumor cells in vivo.

All publications, patents, and patent applications cited in the present specification are hereby incorporated by reference in their entirety.

## Claims

1. An siRNA targeting RecQL1 helicase gene, consisting of:
a sense strand comprising the base sequence of SEQ ID NO: 1, and
an antisense strand comprising the base sequence of SEQ ID NO: 2.

2. The siRNA of claim 1, comprising a natural ribonucleoside, a natural deoxyribonucleoside, and/or a modified nucleoside.

3. The siRNA of claim 2, wherein said modified nucleoside is a 2'-modified nucleoside and/or a bridged nucleoside.

4. The siRNA of claim 3, wherein the 2'-modification group of said 2'-modified nucleoside is a 2'-O-methyl group or a 2'-fluoro group.

5. The siRNA of claim 1, wherein all or part of the internucleoside linkages in said sense strand and/or said antisense strand are a modified internucleoside linkage.

6. The siRNA of claim 1, wherein said antisense strand comprises a 2'-modified nucleoside at position 2 in the base sequence of SEQ ID NO: 2.

7. The siRNA of claim 1, wherein the nucleoside at position 14 in the base sequence of SEQ ID NO: 2 is unmodified in said antisense strand.

8. The siRNA of claim 1, wherein said sense strand and/or said antisense strand comprises 2'-modified nucleoside or a bridged nucleoside at the 3' end.

9. The siRNA of claim 1, wherein:
in said sense strand, the nucleoside at position 7, position 9, and/or position 11 in the base sequence of SEQ ID NO: 1 is unmodified, or is a 2'-fluoro-modified nucleoside; and/or
in said antisense strand, the nucleoside at position 6 and/or position 12 in the base sequence of SEQ ID NO: 2 is unmodified, or is a 2' fluoro-modified nucleoside, and/or the nucleoside at position 7 in the base sequence of SEQ ID NO: 2 is a 2'-O-methyl-modified nucleoside.

10. The siRNA of claim 1, wherein said sense strand and said antisense strand respectively consist of:
(1) the base sequence of SEQ ID NO: 3 and the base sequence of SEQ ID NO: 4,
(2) the base sequence of SEQ ID NO: 5 and the base sequence of SEQ ID NO: 6,
(3) the base sequence of SEQ ID NO: 7 and the base sequence of SEQ ID NO: 8,
(4) the base sequence of SEQ ID NO: 9 and the base sequence of SEQ ID NO: 10, or
(5) the base sequence of SEQ ID NO: 11 and the base sequence of SEQ ID NO: 12.

11. The siRNA of claim 1, wherein each of said sense strand and said antisense strand consists of natural ribonucleosides linked by internucleoside linkages.

12. An agent for inducing cell death, comprising the siRNA of claim 1 as an active ingredient.

13. An agent for treating cancer, comprising the siRNA of claim 1 as an active ingredient.

14. A pharmaceutical composition for treating cancer, comprising the siRNA of claim 1.

15. The pharmaceutical composition of claim 14, wherein said cancer is ovarian cancer, breast cancer, melanoma, gastric cancer, pancreatic cancer, liver cancer, colorectal cancer, lung cancer, head and neck cancer, peritoneal cancer, or cervical cancer.
